# EUROPÄISCHE PATENTSCHRIFT

(11) **EP 2 976 629 B1**
(45) Veröffentlichungstag und Bekanntmachung des Hinweises auf die Patenterteilung: **09.03.2022**
(21) Anmeldenummer: 14700189.5
(22) Anmeldetag: 09.01.2014
(51) Int. Cl.: G01N 27/22, G01N 33/00, F01N 13/00

(54) **SENSORVORRICHTUNG ZUR ERFASSUNG EINER FEUCHTE EINES STRÖMENDEN FLUIDEN MEDIUMS**
SENSOR DEVICE FOR DETECTING A HUMIDITY OF A FLOWING, LIQUID MEDIUM
DISPOSITIF DE DÉTECTION SERVANT À DÉTECTER LA PRÉSENCE D'HUMIDITÉ DANS UN ÉCOULEMENT DE MILIEU FLUIDE

(30) Priorität: 22.03.2013 DE 102013205086
(43) Veröffentlichungstag der Anmeldung: 27.01.2016
(73) Patentinhaber: Robert Bosch GmbH, 70442 Stuttgart (DE)
(72) Erfinder: BRIESE, Achim, 71277 Rutesheim (DE); STEUBER, Frank, 70806 Kornwestheim (DE); KONZELMANN, Uwe, 71679 Asperg (DE); KUENZL, Bernd, 71701 Schwieberdingen (DE); KAUFMANN, Andreas, 89567 Sontheim An Der Brenz (DE)
(86) Internationale Anmeldenummer: PCT/EP2014/050319
(87) Internationale Veröffentlichungsnummer: WO 2014/146799

(56) Entgegenhaltungen:
- EP-A1- 2 439 499
- WO-A1-2008/009330
- WO-A1-2011/070535
- DE-A1-102006 029 631
- DE-A1-102006 060 312
- JP-A- 2000 292 404
- JP-A- 2003 193 830

## Beschreibung

### Stand der Technik

Aus dem Stand der Technik sind zahlreiche Verfahren und Vorrichtungen zur Bestimmung von Strömungseigenschaften fluider Medien, also von Flüssigkeiten oder Gasen, bekannt. Bei den Strömungseigenschaften kann es sich um grundsätzlich beliebige physikalisch und/oder chemisch messbare Eigenschaften handeln, welche eine Strömung des fluiden Mediums qualifizieren oder quantifizieren. Insbesondere kann es sich dabei um die relative oder absolute Feuchte eines Gases oder Gasgemisches handeln. Feuchtesensoren gelangen bekanntermaßen im Ansaugtrakt oder Abgastrakt von Verbrennungskraftmaschinen zum Einsatz. Hierbei ist es beispielsweise zur Regelung der Verbrennungseffizienz, insbesondere für eine rückstandslose und saubere Verbrennung, wichtig, unter anderem die Feuchte der zugeführten Frischluft zu ermitteln, um beispielsweise das entsprechend gewünschte Mischverhältnis zwischen Frischluft und rückgeführtem Abgas einstellen zu können und eine anschließend eine benötigte Menge Treibstoff für eine vorbestimmte Vermischung im Zylinder der Verbrennungskraftmaschine zu bestimmen. Die Information über die Feuchte der zugeführten Umgebungsluft, sowie des Abgases wird vom Motorsteuergerät jedoch für eine Vielzahl an geregelten Prozessen verwendet.

Beispielsweise sind derartige Sensorvorrichtungen zum Einsatz im Ansaugtrakt oder Abgastrakt von Verbrennungskraftmaschinen aus der DE 10 2010 043 062 A1 oder der DE 10 2010 043 083 A1 bekannt. Die gemäß diesen Dokumenten vorgeschlagenen Sensorvorrichtungen stellen eine Kombination mehrerer Sensoren zur Verfügung, um eine Vielzahl an Strömungseigenschaften fluider Medien zu erfassen. Insbesondere ist der DE 10 2010 043 083 A1 hinsichtlich der Ermittlung der Feuchte des fluiden Mediums zu entnehmen, dass in der dort vorgeschlagenen Sensorvorrichtung ein Feuchtesensor oder ein Feuchtemodul aufgenommen ist, welcher zusammen mit der Sensorvorrichtung in einen Strömungskanal eines fluiden Mediums eingebracht wird. Innerhalb des Strömungskanals strömt das gesamte fluide Medium an der semipermeablen Membran des Feuchtesensors vorbei. Hierbei kann es vorkommen, dass auftretende Tropfen aus Kondenswasser an der Kante der um die Membran angeordneten Einlassöffnung hängen bleiben und anschließend die semipermeable Membran benetzen. Die Durchlassfähigkeit der Membran kann hierbei derart beeinträchtigt werden, dass eine zuverlässige Ermittlung der Feuchte des Mediums nicht mehr gewährleistet ist. Ebenso kann durch die unmittelbare Beaufschlagung der Membran mit dem fluiden Medium, welches üblicherweise ein ungefiltertes und/oder mechanisch komprimiertes Prozessgas ist, durch grobe Verunreinigungen wie Staub oder andere Schmutzpartikel in der beispielsweise über den Ansaugtrakt eingesaugten Umgebungsluft beschädigt werden. Dies kann eine direkte Kontamination des Sensorelements und die Zerstörung desselben zur Folge haben.

Weiterhin ist aus der DE 10 2006 033 251 A1 eine Schutzeinrichtung für einen Feuchtesensor bekannt, welche den Sensor vor aggressiven Substanzen in einem zu analysierenden Fluid schützen soll. Die in diesem Dokument vorgeschlagene Schutzeinrichtung sieht eine den Sensor umschließende Kappe vor, welche mit Öffnungen versehen ist, die wiederum mit einer Wasserdampf durchlassenden Membran versehen sind, vor.

Bei vielen derartigen Sensorvorrichtungen, insbesondere bei aktuellen Feuchtesensoren, wird in der Regel mindestens ein Sensorelement verwendet. Dieses mindestens eine Sensorelement wird in der Regel, wie auch in dem oben genannten Stand der Technik beschrieben, durch lediglich eine Schutzmembran vor einem direkten Kontakt mit der Strömung des fluiden Mediums zumindest teilweise geschützt. Eine Kontamination dieser Schutzmembran beispielsweise durch Wasser-Tröpfchen oder Öl-Tröpfchen bedeutet allerdings den zumindest temporären Ausfall der Feuchtemess-Fähigkeit.

### Offenbarung der Erfindung

Es wird dementsprechend eine Sensorvorrichtung nach Anspruch 1 zur Erfassung einer Feuchte eines strömenden fluiden Mediums vorgeschlagen, insbesondere zur Erfassung der Feuchte der Prozessgase im Ansaugtrakt oder Abgastrakt von Verbrennungskraftmaschinen. Bezüglich der möglichen, zu erfassenden Feuchte des Gases, welche als Absolutwert und/oder Relativwert erfasst werden kann, kann auf die obige Beschreibung des Standes der Technik verwiesen werden.

Die Sensorvorrichtung umfasst mindestens ein Feuchtemodul, mit mindestens einem Sensorelement zur Erfassung der Feuchte und mindestens einer das Sensorelement abschirmenden Schutzmembran. Unter einem Sensorelement ist ein Element zu verstehen, welches eingerichtet ist, um mindestens einen Parameter zu erfassen und mindestens ein Signal entsprechend dieser Erfassung des Parameters zu erzeugen. Beispielsweise kann das Sensorelement mindestens einen Sensorchip umfassen, welcher zur Ermittlung der Feuchte eines Gases oder Gasgemisches geeignet ist. Diesbezüglich kann beispielsweise auf die oben im Stand der Technik beschriebenen Feuchtesensoren verwiesen werden. Auch andere Ausgestaltungen des Sensorelements sind jedoch grundsätzlich möglich.

Die Sensorvorrichtung weist weiterhin mindestens ein Gehäuse auf, in welchem ein Messraum angeordnet ist, wobei das Feuchtemodul zumindest teilweise in einem Messraum des Gehäuses angeordnet ist. Unter einem Gehäuse ist allgemein eine Vorrichtung zu verstehen, welche mindestens einen Innenraum umschließt und welche eine Schutzfunktion für eine oder mehrere Komponenten der Sensorvorrichtung ausübt, die in dem mindestens einen Innenraum aufgenommen sind. Insbesondere kann das Gehäuse eingerichtet sein, um eine mechanische Schutzfunktion bereitzustellen. Alternativ oder zusätzlich können auch beispielsweise Schutzfunktionen gegenüber Feuchtigkeit, mechanischen Belastungen beim Verbau der Sensorvorrichtung, thermische Schutzfunktionen oder andere Schutzfunktionen oder Kombinationen der genannten und/oder anderer Schutzfunktionen gegeben sein. Vorzugsweise kann das Gehäuse eingerichtet sein, um eine Schutzwirkung zumindest gegenüber mechanischen Belastungen zu gewährleisten. Beispielsweise kann das Gehäuse zumindest teilweise aus einem steifen Material hergestellt werden, welches beispielsweise bei einem Fixieren des Gehäuses in einem Strömungsraum eines fluiden Mediums bei üblichen Kräften, beispielsweise bei üblichen Verschraubungskräften, keine Verformung durchläuft. Das Gehäuse kann beispielsweise ganz oder teilweise aus einem metallischen Material und/oder einem Kunststoffmaterial hergestellt sein.

Das Gehäuse weist weiter ein in einer axialen Richtung in das strömende fluide Medium hinein ragendes Schutzrohr auf, wobei das Schutzrohr mindestens einen Einströmpfad und mindestens einen Ausströmpfad aufweist. Es handelt sich bei der axialen Richtung um eine Richtung entlang einer durch die Längserstreckung des Schutzrohrs definierten Rotationsachse, welche beispielsweise senkrecht zu einer Längsachse des Strömungsraumes ausgerichtet sein kann. Erfindungsgemäß ist es vorgesehen, dass das fluide Medium durch den Einströmpfad entgegen der axialen Richtung in den Messraum einströmen kann und durch den Ausströmpfad in axialer Richtung aus dem Messraum wieder ausströmen kann.

Das Gehäuse ist derart ausgebildet, dass das fluide Medium entgegen einer axialen Richtung, bevorzugt senkrecht zu der Schutzmembran der Sensorvorrichtung, in den Einströmpfad einströmen kann. Beispielsweise kann diese Achse ebenso im Wesentlichen senkrecht zur Hauptströmungsrichtung des fluiden Mediums ausgerichtet sein. Eine Anordnung mit paralleler oder annähernd paralleler Ausrichtung von Einströmpfad, Ausströmpfad und Schutzmembran ist auch denkbar. Weiter ist es besonders von Vorteil, wenn die Sensorvorrichtung zusätzlich mit ihrer axialen Richtung parallel zu einem Schwerkraftvektor ausgerichtet ist und die Schutzmembran sich im Sinne der Schwerkraftwirkung relativ zur Einlassöffnung und zur Auslassöffnung oben befindet. Das fluide Medium soll derart entgegen der axialen Richtung in den Einströmpfad einströmen können, dass dieses weiter durch den Messraum strömen kann, wo es zu dem Sensorelement gelangt und beispielsweise mit dem Sensorelement und/oder mindestens einer Messoberfläche des Sensorelements in Kontakt gelangen kann und/oder in das Sensorelement einströmen kann, bevor das fluide Medium anschließend in der axialen Richtung durch den Ausströmpfad hindurch zurück in den Strömungsraum strömen kann.

Entsprechend der an das Schutzrohr gestellten Anforderungen, bedarf es in der Regel einer genauen Abstimmung. So muss beispielsweise zur Gewährleistung einer hohen Dynamik des Feuchtesensors in der Regel ein ausreichender durch das Schutzrohr strömender Massestrom erreicht werden, was jedoch wiederum eine erhöhte Strömungsgeschwindigkeit innerhalb des Schutzrohrs und des Messraumes zur Folge haben kann. Hingegen ist das Schutzrohr vorzugsweise derart proportioniert, bzw. bemessen, dass ein Transport von Kondenswasser und Schmutzpartikeln zum Sensorelement minimiert wird, jedoch eine zur Erfassung der Feuchte des Mediums ausreichende Durchströmung des Schutzrohrs gesichert ist. Ein Schutz der Schutzmembran ist insbesondere deshalb erforderlich, um auch eine flächige Benetzung der Schutzmembran mit Wassertropfen oder Öltropfen und insbesondere eine Bildung eines Wasserfilmes zu verhindern, um dadurch einen temporären oder endgültigen Ausfall der Sensorvorrichtung zu verhindern, sowie eine abrasive Wirkung der Schmutzpartikel beim Überströmen der Schutzmembran aufgrund der erhöhten Strömungsgeschwindigkeit zu reduzieren.

Ein weiterer vorteilhafter Effekt eines derartigen Schutzrohrs besteht in einem Fernhalten von Wasser und/oder Schmutzpartikeln in einem Ansaugtrakt oder Abgastrakt von dem Sensorelement und der Schutzmembran, d.h. einem Schutz vor Wasserschlag und/oder Verunreinigung durch angesaugte Staubpartikel, Öltropfen oder Verbrennungsrückstände wie beispielsweise Rußpartikel, wobei gleichzeitig eine ausreichende Überströmung mit dem Medium gewährleistet ist. Folglich kann eine Erfassung der Feuchte des strömenden Mediums mit einer hohen Genauigkeit durchgeführt werden und die Funktionsfähigkeit der Sensorvorrichtung und insbesondere des Sensorelements kann auch in einem stark mit Partikeln, Wassertropfen, Öltropfen oder Öldampf kontaminierten Medium sowie bei einem beispielsweise schwallartigen Ansteigen eines Flüssigkeitsspiegels gewährleistet. Dies ist besonders dann der Fall, wenn die erfindungsgemäße Sensorvorrichtung mit ihrer axialen Richtung parallel zu einem Gravitationsvektor ausgerichtet und in das strömende Medium hineinragt. Mit anderen Worten, wenn die Schutzmembran sich im Sinne der Schwerkraftwirkung relativ zur Einlassöffnung oben befindet.

In bestimmten Fällen, kann sich, beispielsweise nach dem Durchfahren einer Pfütze oder dergleichen, in einem Bereich des Luftansaugtraktes hinter dem Luftfilter eines Fahrzeuges auch eine größere Menge an Wasser sammeln, welche nicht mehr auf anderem Wege abgeleitet werden kann und anschließend durch den hohen Volumenstrom des Mediums mitgerissen werden kann. Dies kann zu einem schwallartigen Beaufschlagen der Sensorvorrichtung führen, bei welcher im Wesentlichen der Einströmpfad und der Ausströmpfad mit einer vergleichsweise großen Menge Wasser in Kontakt gelangt. Im Falle eines Verschließens des Einströmpfades und des Ausströmpfades mit Wasser erzielt das Schutzrohr aufgrund seiner Geometrie eine Schutzwirkung nach Art einer Taucherglocke, gemäß welcher das sich zu diesem Zeitpunkt im Inneren des Schutzrohrs und insbesondere im Inneren des Messraumes befindliche Volumen des Mediums eingeschlossen wird und mit zunehmender Komprimierung durch Erzeugung eines Gegendrucks ein Eintreten des Wassers bis in den Messraum hinein verhindert.

Weiterhin kann es vorteilhaft vorgesehen sein, dass das Schutzrohr erfindungsgemäß eingerichtet, um eine ausreichende Überströmung der Schutzmembran und damit kurze Diffusionszeiten des fluiden Mediums zum Sensorelement sowie eine kurze Reaktionszeit des Sensorelements bezüglich der Erfassung der Feuchte des fluiden Mediums bereitzustellen.

Hierdurch kann sichergestellt werden, dass ein ausreichender Austausch des im Schutzrohr befindlichen Mediums erfolgt, um eine genaue Messung der Feuchte zu ermöglichen. Der Austausch des im Schutzrohr befindlichen Mediums erfolgt bevorzugt mit einer hohen Geschwindigkeit, um in vorteilhafter Weise eine zeitliche Verzögerung zwischen einer Änderung der Feuchte des strömenden Mediums und der Erfassung dieser Änderung durch das Sensorelement zu minimieren.

Nach einer weiteren Ausgestaltung der Erfindung, kann es vorgesehen sein, dass der Einströmpfad und der Ausströmpfad innerhalb des Schutzrohrs rotationssymmetrisch zu einer in der axialen Richtung verlaufenden Rotationsachse ausgebildet sind. Hierdurch wird zunächst eine vorteilhafte Ausgestaltung des Schutzrohrs als Rotationskörper geschaffen, der sich in einfacher Weise mit geringem Aufwand produzieren lässt. Eine Vermeidung jeglicher Ecken und Kanten auf der bevorzugt rotationssymmetrischen Außenfläche des Schutzrohrs, hat weiterhin den Vorteil, dass dieser einen geringeren Strömungswiderstand im fluiden Medium aufweist und zudem eine Verwirbelung des Fluides in höchstem Maße vermieden wird.

Nach der zuletzt beschriebenen Ausführungsform der erfindungsgemäßen Sensorvorrichtung ist es weiterhin von Vorteil, wenn das Sensorelement des Feuchtemoduls fluchtend zur Rotationsachse angeordnet ist und somit von der zu analysierenden Strömung des durch das Schutzrohr hindurch strömenden Fluides unmittelbar beaufschlagt wird. Hierdurch ist ebenfalls in vorteilhafter Weise eine gleichmäßige Beaufschlagung des Sensorelements auch bei pulsierender Strömung, sowie bei einer Strömungsumkehr gegeben. Zu einer Steigerung des Massenstromes über die Schutzmembran und damit auch über das Sensorelement, ist ebenfalls eine asymmetrische Ausrichtung des Sensorelements relativ zu der Rotationsachse des Schutzrohrs denkbar.

Besonders vorteilhaft ist es ferner, wenn die axiale Richtung des Schutzrohrs im Wesentlichen senkrecht zur Strömungsrichtung des fluiden Mediums und zusätzlich parallel zu einem Schwerkraftvektor ausgerichtet ist. In meist rohrförmig ausgestalteten Strömungsräumen oder Strömungskanälen, wie sie üblicherweise auch im Ansaugtrakt oder Abgastrakt von Verbrennungskraftmaschinen zum Einsatz gelangen, lässt sich das Schutzrohr somit auf besonders einfache Weise im Wandbereich des Strömungskanals befestigen. Eine Ausrichtung des Schutzrohrs senkrecht zur Hauptströmungsrichtung des fluiden Mediums innerhalb des Strömungsraumes schafft weiterhin den Vorteil, dass unabhängig von der rotatorischen Ausrichtung des Schutzrohrs eine gleichbleibende Anströmfläche gebildet wird. Zudem erfolgt im Bereich des Schutzrohrs eine Umlenkung eines Teilstroms des strömenden Mediums um annähernd 90°, in Richtung des Einströmpfades. Durch diese hohe Änderung der Strömungsrichtung wird ebenfalls ein Eindringen von beispielsweise Wassertropfen in das Schutzrohr verhindert, da dieser beispielsweise aufgrund seiner Massenträgheit dieser abrupten Richtungsänderung nicht folgen kann.

Weiter ist vorgesehen, dass der Einströmpfad den Ausströmpfad zumindest teilweise konzentrisch ringförmig umschließt. Hierdurch wird eine homogenere Anströmung der Schutzmembran im Messraum, der den Einströmpfad und den Ausströmpfad verbindet, bewirkt.

Ebenso kann eine Ausgestaltung des Schutzrohrs vorgesehen sein, bei der der Einströmpfad mindestens eine Einlassöffnung im Schutzrohr aufweist und der Ausströmpfad mindestens eine Auslassöffnung im Schutzrohr aufweist. Hierbei ragt das Schutzrohr bevorzugt derart in eine Strömung des fluiden Mediums hinein, dass die Auslassöffnung weiter im Inneren der Strömung angeordnet ist als die Einlassöffnung. Das bevorzugt senkrecht in den Strömungsraum des fluiden Mediums hineinragende Schutzrohr bietet dem fluiden Medium durch die mit der Außenfläche des Schutzrohrs gebildete Angriffsfläche somit einen Strömungswiderstand, insbesondere durch die Außenfläche des gegenüber dem Gehäuse im Bereich des Ausströmpfades verlängerten Ausströmstutzens und des somit folglich weiter in das Innere des Strömungsraums hineinragenden Ausströmstutzen. Die angeströmte Außenfläche des Schutzrohrs bewirkt, dass das anströmende fluide Medium auf seiner strömungsaufwärtigen Seite gestaut wird, was wiederum einen Druckanstieg in diesem Bereich, also im Bereich der mindestens einen Einlassöffnung zur Folge hat. Zudem kann durch Abrisskanten unmittelbar an der mindestens einen Einlassöffnung sichergestellt werden, dass unmittelbar an der Einlassöffnung ein Bereich relativ niedriger Geschwindigkeiten und gemäß des Bernoulli-Effektes relativ hohen statischen Druckes entsteht. Durch den Verdrängungseffekt des gesamten Schutzrohres und insbesondere durch die Anbringung eines relativ großen Radius an der Auslassöffnung am Unterdruckstutzen wird im Bereich der Auslassöffnung eine relativ hohe Strömungsgeschwindigkeit des Mediums bewirkt. Auf diese Weise kann insbesondere sichergestellt werden, dass an der Auslassöffnung infolge des Bernoulli-Effekts ein geringerer statischer Druck herrscht als im Bereich der Einlassöffnung, was für einen Kamineffekt und somit eine Durchströmung des Schutzrohrs in der erfindungsgemäß beabsichtigten Richtung von der mindestens einen Einlassöffnung zu der Auslassöffnung führt. Zur genauen Einstellung der Durchströmung des Schutzrohrs können die Einlassöffnungen einen weiter angepassten Öffnungsquerschnitt aufweisen, welcher sowohl eine kreisförmige, ovale oder eine andere geometrisch günstige Form aufweisen kann. Hierdurch kann insbesondere die Reaktionszeit und die damit einhergehende Messgenauigkeit der Sensorvorrichtung beeinflusst werden.

Gemäß der zuvor beschriebenen Ausführungsform, kann es weiterhin von Vorteil sein, wenn eine Mehrzahl von konzentrisch um die Auslassöffnung radial beabstandet angeordneten Einlassöffnungen vorgesehen ist. Beispielsweise weist das Schutzrohr mindestens einen Einströmstutzen und bevorzugt mehrere Einstömstutzen auf. Dies trägt insbesondere dem Gedanken der rotationssymmetrischen Ausgestaltung des Schutzrohrs Rechnung, welche auf besonders vorteilhafte Weise eine Abhängigkeit der Effektivität der Sensorvorrichtung zu der rotatorischen Ausrichtung bei der Montage des Schutzrohrs in dem Strömungsraum minimiert. Die Mehrzahl an Einlassöffnungen ist hierbei bevorzugt äquidistant zur Rotationsachse des Schutzrohrs und weiter in einem vorgegebenen und gleichbleibenden Winkelabstand zueinander angeordnet. Das bedeutet, dass jede der Einlassöffnungen entlang einer gedachten Kreislinie um die Rotationsachse folgend, den immer gleichen Abstand zu den jeweils benachbarten Einlassöffnungen aufweist. Diese bedingte winkeldiskrete Anordnung der Einlassöffnungen hat im individuellen Einsatz für jede denkbare Rotations-Winkelstellung des Schutzrohrs eine geringfügige Schwankung der Durchströmung des Schutzrohrs und damit auch in den messtechnischen Eigenschaften der Sensorvorrichtung, jedoch kann diese geringe Schwankung der Durchströmung beispielsweise durch eine entsprechende Anpassung der Öffnungsquerschnitte der Einlassöffnungen teilweise oder gänzlich ausgeglichen werden.

Weiter ist der Ausströmpfad zumindest teilweise in dem Ausströmstutzen innerhalb des Schutzrohrs angeordnet sein. Jeweils eine Einlassöffnung kann in jeweils einem Einströmstutzen angeordnet sein. Als Ausströmstutzen bzw. Einströmstutzen wird insbesondere ein Element verstanden, welches zumindest abschnittsweise rohrförmig gestaltet ist, beispielsweise als Hohlzylinder. Aufgrund der herrschenden Druckverhältnisse an der Auslassöffnung des Ausströmstutzens, kann dieser auch als Unterdruckstutzen bezeichnet werden. Hinsichtlich einer nachfolgend beschriebenen Ausgestaltung des Ausströmstutzens wird sich ausdrücklich nicht auf rohrförmige Elemente mit konstantem Innen- oder Außendurchmesser beschränkt, dieser kann insbesondere variieren. Der Ausströmstutzen kann bevorzugt eine Wandstärke von ca. 0,2 mm bis 10 mm und weiter bevorzugt eine Einbautiefe von 5 mm bis 50 mm aufweisen. Ebenso kann es vorgesehen sein, dass der Ausströmstutzen einen Innendurchmesser bevorzugt im Bereich von ca. 0,5 mm bis 10 mm und einen Außendurchmesser bevorzugt im Bereich von ca. 1 mm bis 20 mm aufweist. Weiter weist der Ausströmpfad bevorzugt annähernd die gleiche Querschnittsfläche auf, wie die Summe der Querschnitte der relevanten Einlassöffnungen. Zu den relevanten Einlassöffnungen werden jene Einlassöffnungen gezählt, welche nach der Montage des Schutzrohrs insbesondere entgegen der Hauptströmungsrichtung des Mediums ausgerichtet sind sowie auch diejenigen, die stromabwärts, also in Richtung der Hauptströmungsrichtung des Mediums ausgerichtet sind und in der erfindungsgemäß beabsichtigen Weise aufgrund der relativ hohen statischen Drücke zu der Durchströmung des Schutzrohrs und damit des Messraumes beitragen.

Somit kann, wenn der Ausströmpfad an seinem der Sensorvorrichtung zugewandten Ende eine Querschnittsverjüngung aufweist, ferner die vorteilhafte Wirkung einer Verstärkung des Bernoulli-Effekts bewirkt werden, weil dadurch die Überströmungsgeschwindigkeit im Bereich der Schutzmembran gesteigert und damit die Antwortzeit des Sensorelements verringert wird. Als Querschnittsverjüngung wird allgemein verstanden, dass das Schutzrohr in einem bestimmten Abschnitt einen geringeren Querschnitt aufweist, als in einem daran angrenzenden Abschnitt. Insbesondere eine diffusorartige Ausgestaltung der Innenseite, bzw. der Innenfläche des Ausströmstutzens, welche durch eine kontinuierliche Querschnittsverjüngung entgegen der Strömungsrichtung im Ausströmpfad, bzw. einer Querschnittserweiterung in Strömungsrichtung im Ausströmpfad betrachtet gebildet ist, schafft eine besonders vorteilhafte Verstärkung des Bernoulli-Effekts, also eine Geschwindigkeitserhöhung im Bereich der Membran. Ein diffusorartig ausgebildeter Ausströmpfad bewirkt geringere Wandschubspannungen im Ausströmstutzen. Mit anderen Worten, also einen geringeren Druckverlust und eine niedrige Austrittsgeschwindigkeit des durch das Schutzrohr strömenden Mediums aufgrund des großen Querschnittes der Auslassöffnung. Damit wird eine lokal effektive Nutzung dieses Unterdruck-Niveaus erreicht. Denkbar ist auch eine lediglich abschnittsweise diffusorartige Ausgestaltung beispielsweise am Ende des Ausströmpfads, im Bereich der Auslassöffnung.

Des Weiteren ist zwischen der Mündung des Einströmpfads in den Messraum und der Mündung des Ausströmpfads in den Messraum mindestens ein zur Schutzmembran parallel verlaufender Konturabschnitt angeordnet ist, um in vorteilhafter Weise ein möglichst paralleles Vorbeiströmen des fluiden Mediums an der Schutzmembran des Sensorelements zu bewirken. Ebenso kann es hierbei vorgesehen sein, dass das Schutzrohr im Bereich des Einströmpfads und des Ausströmpfads einen Radius im Bereich von bevorzugt 0,1 mm bis 10 mm, besonders bevorzugt im Bereich von 0,5 mm bis 10 mm aufweist.

Weiterhin ist es denkbar, dass, zur Steigerung des Massenstromes über die Schutzmembran uns damit auch über das Sensorelement, eine asymmetrische Ausrichtung des Sensorelements relativ zu der Rotationsachse des Schutzrohrs vorgesehen ist. Bei einer derartigen asymmetrischen Variante existiert ein Versatz des Sensorelements zur Rotationsachse des Schutzrohrs entgegen Hauptströmungsrichtung, insbesondere eine Ausrichtung des Sensorelements parallel zur Rotationsachse in Richtung und bis auf Höhe des stromaufwärtigen Konturabschnitts. Damit wird auf vorteilhafte Weise eine Erhöhung der Dynamik des erfassten Feuchte-Signals bewirkt. Ergänzend zu der zuvor beschriebenen Ausführung der Sensorvorrichtung ergibt sich der Vorteil, dass die im Bereich des Konturabschnitts einstellende parallele Vorbeiströmung des Mediums im Bereich des bevorzugt mittig hinter der Schutzmembran angeordneten Sensorelements erfolgt.

Am Schutzrohr ist ferner zwischen der äußeren Umfangsfläche des Ausströmstutzens und der Einlassöffnungen einen Ringspalt vorgesehen sein, um den sogenannten "Splash-Effekt", sprich das vereinzelte Spritzen von auf der Außenfläche des Schutzrohres aufprallenden Wassertropfen in Richtung der Einlassöffnungen, besonders vorteilhaft zu verhindern. Hierbei ist es ebenso von Vorteil, wenn die Abmessungen des Abstandes und der Durchmesser der Einlassöffnungen zum Schutzrohr derart gewählt sind, dass die durch den Unterdruck im Bereich der Einlassöffnungen auftretende Sogwirkung keine herumspritzenden Tropfen eingesaugt werden.

Im Bereich der einen oder mehreren Einlassöffnungen kann weiterhin wenigstens eine Abrisskante gebildet sein, wodurch ein möglicherweise vom fluiden Medium mitgetragener Tropfen aus Kondenswasser oder ein von der Rohrwand in Richtung der Einlassöffnung fließender Wasserfilm an der Abrisskante zunächst hängenbleibt und erneut in Tropfenform vom vorbeiströmenden Medium mitgerissen wird, ohne in die Einlassöffnung einzutreten. Auch ein an der Außenfläche des Schutzrohrs entlang laufender Tropfen aus Kondenswasser kann somit vom Eintritt in den Einströmpfad gehindert werden, um in vorteilhafter Weise ein Kontakt desselben mit der Schutzmembran des Sensormoduls zu verhindern. Weiterhin kann eine Abrisskante im äußeren Bereich des Gehäuses, insbesondere durch eine Kante des Gehäuses im Bereich der Dichtung gebildet sein, welche nach der Montage der Sensorvorrichtung aus der Ebene des Wandabschnitts des Strömungsraums herausragt.

Nach einer weiteren Ausführungsform der Erfindung, wird durch eine radial außerhalb der einen oder mehreren Einlassöffnungen vorgesehenen Rippe, welche einen umfänglichen Ableitkanal bildet, in vorteilhafter Weise unter Nutzung einer auftretenden Kapillarwirkung ein von der Rippe abgefangener und in den Ableitkanal geleiteter Tropfen um die Einlassöffnungen herum auf die strömungsabwärts seitige Außenfläche des Schutzrohrs geführt. Ebenso wird ein sind aufgrund von Temperaturunterschieden im Wandbereich des Strömungsraums bildender Film aus Kondenswasser von der Rippe abgefangen. Auf diesem Wege wird ein mögliches Eintreten eines Wassertropfens in den Einströmpfad und einer Beaufschlagung der Schutzmembran mit dem Wassertropfen verhindert.

Eine weiterhin vorteilhafte Ausführungsform sieht es vor, dass das Gehäuse mindestens ein Befestigungselement zum Befestigen des Gehäuses an einer Wand eines von dem fluiden Medium durchströmten Strömungsraums oder Strömungskanals aufweist, wobei das Befestigungselement derart ausgestaltet ist, dass in befestigtem Zustand das Schutzrohr zumindest teilweise und bevorzugt mit einer Einbautiefe von bis zu 50 mm in den Strömungsraum hineinragt. Der Strömungsraum weist hierbei bevorzugt einen im Wesentlichen runden oder ovalen Querschnitt auf. Ein rechteckiger, bzw. ein mit wenigstens einem ebenen Abschnitt versehener Querschnitt ist jedoch ebenfalls denkbar. Das Gehäuse ist bevorzugt derart mit dem Strömungsraum, insbesondere mit der Außenwand des Strömungsraumes, verbindbar, dass in befestigtem Zustand das Schutzrohr des Gehäuses durch eine in einem Wandabschnitt des Strömungsraumes vorgesehene Öffnung hindurch zumindest teilweise in den Strömungsraum hineinragt. Zu diesem Zweck kann das Gehäuse beispielsweise mindestens ein Fixierelement umfassen, welches eingerichtet ist, um eine mechanische Fixierung und/oder Stabilisierung des Gehäuses relativ zu dem Strömungsraum zu bewirken. Das Gehäuse kann hierzu beispielsweise aus einem metallischen Material gefertigt sein. Alternativ oder zusätzlich können jedoch auch andere Materialien verwendet werden, beispielsweise keramische Materialien und/oder Kunststoffmaterialien.

Zur Befestigung der Sensorvorrichtung in dem Wandabschnitt des Strömungsraumes, ist es weiterhin von Vorteil, wenn das Befestigungselement mindestens ein Dichtelement aufweist, insbesondere mindestens einen das Schutzrohr umschließenden O-Ring, um ein gasdichtes Verschließen des Strömungsraumes im Bereich der Sensorvorrichtung sicherzustellen. Hierdurch kann ein Austausch von dem zu analysierenden Medium mit der außerhalb des Strömungsraums befindlichen Umgebungsatmosphäre verhindert werden.

Eine zugleich vorteilhafte Ausgestaltung der erfindungsgemäßen Sensorvorrichtung kann es sein, wenn das Gehäuse zumindest teilweise als Steckfühler ausgestaltet ist. Alternativ ist auch eine Ausgestaltung des Gehäuses in Form einer Zwischenplatte oder Sensoraufnahmeplatte vorgenommen werden, welche möglicherweise bei bestehenden Sensoren, gegebenenfalls mit geringfügigen mechanischen Änderungen, mit wenigen Handgriffen nachgerüstet werden kann.

Nach einer weiteren Ausführungsform der Erfindung, kann im Gehäuse mindestens ein Elektronikraum gebildet sein. Weiterhin ist mindestens eine Ansteuerschaltung vorgesehen, welche zur Steuerung des Feuchtesensors, sowie zur Weiterleitung eines erzeugten Messsignals vorgesehen ist. Die Ansteuerschaltung der Sensorvorrichtung kann weiterhin ebenso eine Auswerteschaltung mit umfassen und kann bevorzugt im Elektronikraum angeordnet sein. Ein weiterer Vorteil ist dadurch gegeben, wenn das Feuchtemodul zumindest teilweise in dem Elektronikraum angeordnet ist, wobei der Elektronikraum bevorzugt zumindest teilweise außerhalb des fluiden Mediums angeordnet ist. Unter einem Elektronikraum ist ein Raum zur Aufnahme einer elektronischen Schaltung oder von elektronischen Bauteilen zu verstehen, welcher bevorzugt als ein quaderförmiger Hohlraum gestaltet ist. Er kann jedoch auch ein eine bevorzugt rechteckige Grundfläche aufweisender Halbzylinder sein.

### Kurze Beschreibung der Zeichnungen

Ausführungsbeispiele der Erfindung sind in den Figuren dargestellt und werden nachfolgend exemplarisch näher erläutert.

Es zeigen:
- Fig. 1: eine Querschnittsdarstellung einer bevorzugten Ausführungsform der erfindungsgemäßen Sensorvorrichtung,
- Fig. 1A: eine vergrößerte Ansicht des Details A der Figur 1,
- Fig. 1B: eine vergrößerte Ansicht des Details B der Figur 1,
- Fig. 2: eine perspektivische Schnittdarstellung einer bevorzugten Ausführungsform der erfindungsgemäßen Sensorvorrichtung
und
- Fig. 3: eine Querschnittsdarstellung einer weiteren Ausführungsform der erfindungsgemäßen Sensorvorrichtung.

### Ausführungsformen der Erfindung

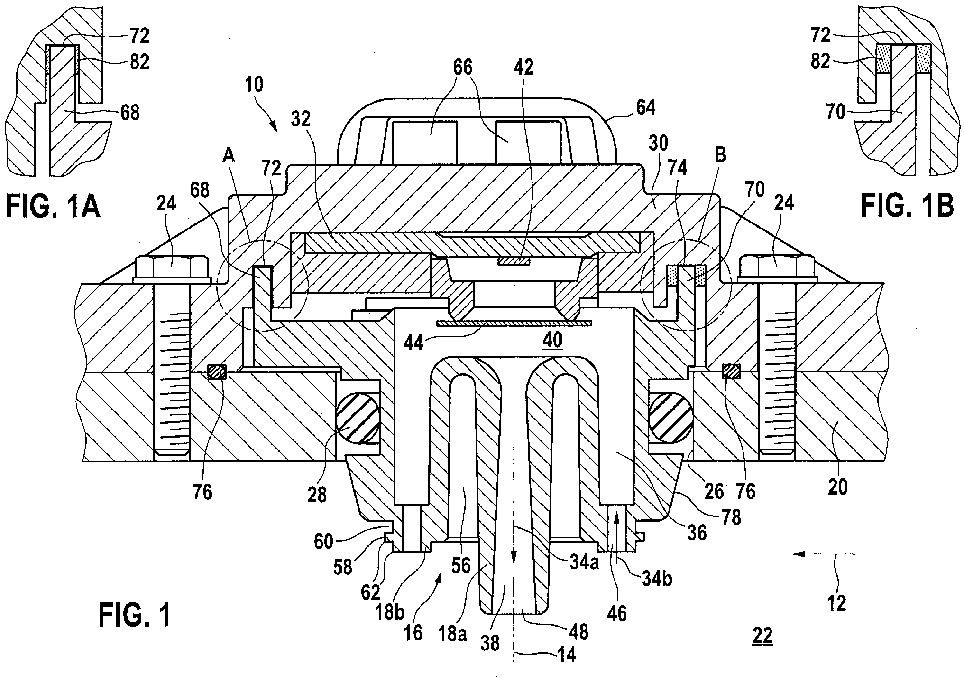

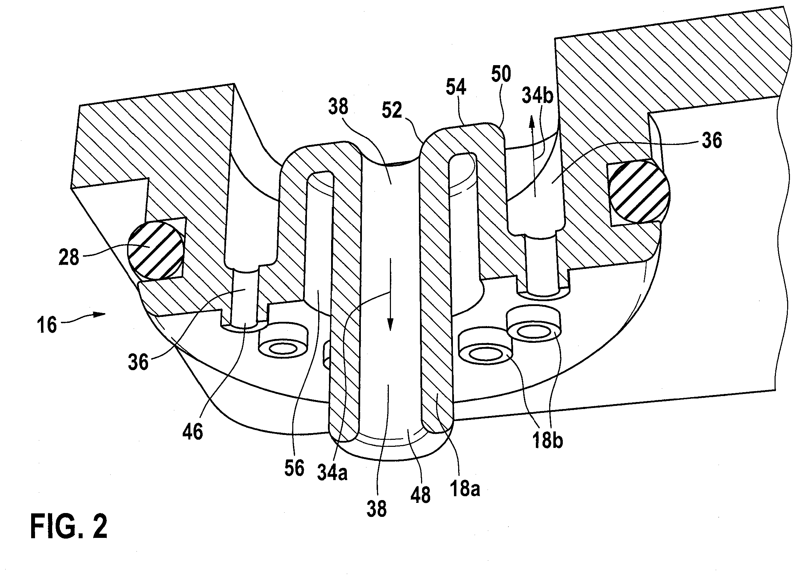

In den Figuren 1 und 2 sind zwei sich lediglich geringfügig unterscheidende Ausführungsformen der erfindungsgemäßen Sensorvorrichtung 10 in unterschiedlichen Blickwinkeln dargestellt. Die Darstellung der Figur 1 zeigt die Sensorvorrichtung 10 mit einem auf dem Schutzrohr 16 angeordneten Feuchtemodul 32. In Figur 2 ist eine alternative Ausführungsform des Schutzrohrs 16 dargestellt, bei der zur Vereinfachung und zur Erläuterung das Feuchtemodul 32 nicht mitdargestellt ist. Die beiden Darstellungen werden im Folgenden gemeinsam beschrieben.

Figur 1 zeigt eine Schnittdarstellung einer bevorzugten Ausführungsform der Sensorvorrichtung 10, wobei der Schnitt in einer Ebene parallel zu einer Hauptströmungsrichtung 12 des fluiden Mediums und entlang einer Rotationsachse 14 des Schutzrohrs 16 verläuft. Das Schutzrohr 16 umfasst einen Ausströmstutzen 18a und mindestens einen Einströmstutzen 18b. Bevorzugt umfasst das Schutzrohr 16 mehrere Einströmstutzen 18b, die konzentrisch zu dem Ausströmstutzen 18a angeordnet sind, wie in Figur 1 gezeigt ist. Beispielsweise ist der Ausströmstutzen 18a koaxial zu der Rotationsachse 14 angeordnet und sind die Einströmstutzen 18b in einer Umfangsrichtung um die Rotationsachse 14 gleichmäßig verteilt radial außerhalb des Ausströmstutzens 18a angeordnet. Der Ausströmstutzen 18a wirkt als Unterdruckstutzen und die Einströmstutzen 18b wirken als Überdruckstutzen wie nachstehend ausführlicher beschrieben wird. Ein die Hauptströmungsrichtung 12 kennzeichnender Pfeil würde in der Bildebene der Figur 1 liegen. Aufgrund des bevorzugt rotationssymmetrischen Aufbaus des Schutzrohrs 16 kann zur leichteren Beschreibung eine im Bild von rechts nach links verlaufende Hauptströmungsrichtung 12 des fluiden Mediums angenommen werden.

Des Weiteren kann der in Figur 1 gezeigten Ausführungsform im Detail entnommen werden, dass die erfindungsgemäße Sensorvorrichtung 10 in einem Wandabschnitt 20 eines Strömungsraumes 22 angeordnet ist und durch die Befestigungselemente 24 an dem Wandabschnitt 20 gehalten ist. Hierbei ist der Darstellung insbesondere zu entnehmen, dass das Schutzrohr 16 der gezeigten Ausführungsform der Sensorvorrichtung 10 in montiertem Zustand durch eine Durchtrittsöffnung 26 im Wandabschnitt 20 in den Strömungsraum 22 hineinragt. Das Schutzrohr 16 weist hierbei eine nicht näher bezeichnete Nut zur Aufnahme eines Dichtelements 28, welches beispielsweise ein O-Ring sein kann, auf. Das Dichtelement 28 dient einerseits als Lagerung des Schutzrohrs 16 in der Durchtrittsöffnung 26, und andererseits zur gasundurchlässigen Abdichtung der Durchtrittsöffnung 26 gegenüber der äußeren Umgebung.

Ferner kann der Darstellung von Figur 1 entnommen werden, dass die erfindungsgemäße Sensorvorrichtung 10 ein Gehäuse 30 aufweist, in welchem ein Feuchtemodul 32 zumindest teilweise angeordnet ist. Weiterhin weist das Gehäuse 30 der dargestellten bevorzugten Ausführungsform der Sensorvorrichtung 10 ein in einer axialen Richtung 34a in das strömende fluide Medium ragendes Schutzrohr 16 auf. In dem Schutzrohr 16 sind wiederum mindestens ein Einströmpfad 36 sowie ein Ausströmpfad 38 gebildet. Der Ausströmpfad 38 im Schutzrohr 16 ist hierbei bevorzugt rotationssymmetrisch um die Rotationsachse 14 angeordnet. Der Einströmpfad 36 ist ferner corotationssymmetrisch um die Rotationsachse 14 und den Ausströmpfad 38 gebildet. Genauer ist der Ausströmpfad 38 in dem Ausströmstutzen 18a ausgebildet und jeweils ein Einströmpfad 36 ist in einem der Einströmstutzen 18b ausgebildet.

Wie der Darstellung von Figur 1 dabei weiter entnommen werden kann, steht der Einströmpfad 36 und der Ausströmpfad 38 über einem Messraum 40 strömungsmäßig in Verbindung. Auf der von dem Ausströmpfad 38 aus betrachteten gegenüberliegenden Ende des Messraums 40 ist koaxial fluchtend zum Ausströmpfad 38 das Sensorelement 42 des Feuchtemoduls 32 angeordnet. Wie der Darstellung von Figur 1 hierbei im Detail entnommen werden kann, ist das Sensorelement 42 des Feuchtemoduls 32 räumlich durch eine Schutzmembran 44 von dem Messraum 40 getrennt.

Die Wirkweise des erfindungsgemäßen Schutzrohrs 16 lässt sich insbesondere dadurch erklären, dass das im Wesentlichen senkrecht zur Rotationsachse 14 im Strömungsraum 22 vorbeiströmende fluide Medium an der strömungsaufwärts-seitigen Außenfläche des Ausströmstutzens 18a gestaut wird und die Strömungsgeschwindigkeit in diesem Bereich herabgesetzt wird. Das fluide Medium strömt demnach im Bereich der Einströmstutzen 18b, insbesondere der Einlassöffnung 46 mit einer geringeren Geschwindigkeit als das Medium im Bereich der Auslassöffnung 48 am unteren Ende des Ausströmstutzens 18a. Entsprechend der somit höheren Strömungsgeschwindigkeit an der Auslassöffnung 48 herrscht in diesem Bereich ebenfalls ein höherer dynamischer Druck als im Bereich der Einlassöffnung 46. Physikalisch betrachtet verhält sich die Strömungsgeschwindigkeit proportional zum dynamischen Druck des fluiden Mediums, welcher sich wiederum antiproportional zum statischen Druck verhält. Folglich wird hierbei durch den Strömungswiderstand, den der Ausströmstutzen 18a darstellt ein Bereich vor der Einlassöffnung 46 geschaffen, in welchem ein höherer statischer Druck herrscht als im Bereich der Auslassöffnung 48. Da das fluide Medium hinsichtlich dieser Druckdifferenzen ein natürliches Ausgleichsbestreben hat, strömt ein Teil des fluiden Mediums durch die Einlassöffnung 46 entlang des Einströmpfads 36 hin zum Messraum 40. Von dem Messraum 40 aus strömt das fluide Medium weiter in axialer Richtung 34a entlang des Ausströmpfades 38 und über die Auslassöffnung 48 zurück in den Strömungsraum 22.

Zur weiteren Abdichtung des Messraumes 40 gegenüber der äußeren Umgebung ist gemäß der dargestellten Ausführungsform bevorzugt ein weiteres Dichtelement 76 vorgesehen, welches beispielsweise durch eine bekannte O-Ring-Dichtung ausgebildet sein kann und in einem Bereich um die Durchführungsöffnung 26 verlaufend zwischen dem Wandabschnitt 20 und dem Gehäuse 30 angeordnet ist. Das Schutzrohr 16 weist wie dargestellt bevorzugt weiter einen Lagerzapfen 68 sowie mindestens einen weiteren Lagerzapfen 70 auf, mit welchen sich das Schutzrohr 16 gegenüber dem Gehäuse 30 abstützt. Das Gehäuse 30 weist hierzu bevorzugt eine Ausrichtebohrung 72, welche bevorzugt zur Aufnahme des Lagerzapfens 68 dient, sowie eine Lagerbohrung 74 zur Aufnahme des weiteren Lagerzapfens 70, auf. Die Ausrichtebohrung 72 weist hierbei bevorzugt einen Innendurchmesser aus, der im Wesentlichen dem Außendurchmesser des Lagerzapfens 68 entspricht. Bevorzugt wird also mit anderen Worten eine Ausbildung für das Schutzrohr 16 und das Gehäuse 30, bei der die Ausrichtebohrung 72 und der Lagerzapfen 68 mit engen Toleranzen gefertigt sind. Das Spiel zwischen dem Lagerzapfen 68 und der Ausrichtebohrung 72 wird folglich soweit wie technisch bzw. fertigungstechnisch möglich gering ausgelegt. Hierdurch wird zusätzlich zu der stirnseitigen Anlagefläche des Lagerzapfens 68 in der Ausrichtebohrung 72 eine vollumfängliche Anlagefläche geschaffen, die also neben der Strinseite des Lagerzapfens 68 auch zumindest einen Teil seiner Umfangsfläche umfasst, wodurch eine seitliche Führung des Lagerzapfens 68 und damit auch des Schutzrohrs 16 erreicht wird. Hierdurch lässt sich das Schutzrohr 16 weiterhin relativ zum Sensorelement 42 in einer Richtung senkrecht, bevorzugt in Hauptströmungsrichtung seitlich, zur Rotationsachse 14 ausrichten. Der weitere Lagerzapfen 70 ist in der Lagerbohrung 74 bezüglich einer entsprechenden seitlichen Ausrichtung frei beweglich angeordnet und stützt sich lediglich mit einer stirnseitigen Kontaktfläche gegenüber dem Gehäuse 30 ab.

Figur 1A zeigt eine vergrößerte Ansicht des Details A der Figur 1 und Figur 1B zeigt eine vergrößerte Ansicht des Details B der Figur 1. Zur Fixierung des Schutzrohrs 16 an dem Gehäuse 30 kann beispielsweise in die Ausrichtebohrung 72 und/oder die Lagerbohrung 74 ein Klebstoff 82 eingebracht werden, der dann mit der Außenumfangsfläche des Lagerzapfens 68 und/oder des weiteren Lagerzapfens 70 verklebt, wie in Figur 1A und 1B dargestellt ist. Beispielsweise wird der Klebstoff 82 so in die Ausrichtebohrung 72 und/oder die Lagerbohrung 74 eingebracht, dass dieser das stirnseitige Ende des Lagerzapfens 68 und/oder des weiteren Lagerzapfens 70 sowie zumindest einen Teil der Außenumfangsfläche des Lagerzapfens 68 und/oder des weiteren Lagerzapfens 70 und die umgebenden Wandabschnitte der Ausrichtebohrung 72 und/oder der Lagerbohrung 74 berührt.

Das Schutzrohr 16 liegt weiter bevorzugt über eine nicht bezeichnete Fläche auf der Außenseite des Wandabschnitts 20 an. Somit wird das Schutzrohr 16 zwischen dem Gehäuse 30 und dem Wandabschnitt 20 durch ein Einklemmen des Schutzrohrs 16 gehalten. Zur Erzielung eines beispielsweise seitlichen Versatzes des Sensorelements 42 zur Rotationsachse 14 des Schutzrohrs 16, kann beispielsweise die Ausrichtebohrung 72 im Gehäuse 30 entsprechend positioniert werden, dass der gewünschte Versatz erreicht wird. Gegebenenfalls kann es hierzu ebenfalls erforderlich sein, die nicht näher bezeichneten Bohrungen für die Befestigungselements 24 im Gehäuse 30 ebenso mit einem entsprechenden Versatz vorzusehen.

Weiterhin ist der Darstellung von Fig. 1 zu entnehmen, dass über die Länge der beiden Lagerzapfen 68, 70 die Möglichkeit besteht das Gehäuse 30 in und entgegen der Richtung entlang der Rotationsachse 14 relativ zum Schutzrohr 16 auszurichten. Insbesondere lässt sich hierdurch die Höhe des Messraums 40 anpassen. Dies beeinflusst folglich weiter die Durchströmung des Messraums 40 und damit ebenso die Reaktionszeiten und die Empfindlichkeit der Sensorvorrichtung 10, wodurch in vorteilhafter Weise eine schnellere und genauere Erfassung der Feuchte des Mediums ermöglicht wird.

Gemäß der in Figur 2 gezeigten Darstellung einer bevorzugten Ausführungsform der Erfindung kann es vorgesehen sein, dass in dem Bereich der Mündung 50 des Einströmpfads 36 in den Messraum 40 sowie der Mündung 52 des Ausströmpfads 38 in den Messraum 40 ein Konturabschnitt 54 vorgesehen ist, welcher den Einströmpfad 36 und den Ausströmpfad 38 verbindet. Der Konturabschnitt 54 kann hierbei bevorzugt einen Radius, bzw. zwei Radien aufweisen, dass zwischen der Mündung 50 des Einströmpfads 36 und der Mündung 52 des Ausströmpfads 38 ein ebener, der Schutzmembran 44 parallel verlaufender Konturabschnitt 54 gebildet ist. Dieser bevorzugt im Messraum 40 abschnittsweise parallel zur Schutzmembran 44 verlaufende Konturabschnitt 54, bewirkt eine ebenso abschnittsweise parallel zur Schutzmembran 44 gerichtete Strömung des fluiden Mediums, was sich vorteilhaft auf den Messprozess auswirkt.

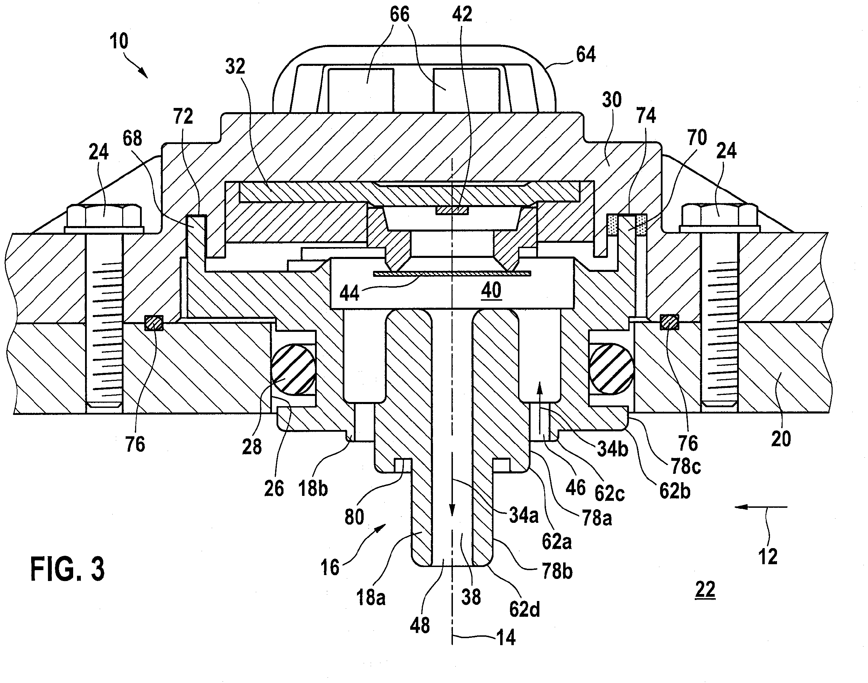

Um erfindungsgemäß ein Eindringen von in der Strömung des fluiden Mediums mitgetragenen Wassertropfen, welche sich üblicherweise im Größenbereich der Einlassöffnung 46 bewegen können, zu verhindern, ist wie in der Darstellung von Figur 1 gezeigt bevorzugt ein Ringspalt 56 zwischen dem Einströmpfad 36 und Ausströmpfad 38 angeordnet. Hierdurch wird ein Einsaugen oder Eindringen von kleineren Wassertröpfchen, welche durch einen Aufprall eines größeren Wassertropfens auf der Außenfläche des Ausströmstutzens 18a durch entsprechendes seitliches Wegspritzen entstehen, in vorteilhafter Weise verhindert, da diese in den hierfür vorgesehenen Ringspalt 56 hineinspritzen bzw. wie in Fig. 3 zu erkennen aufgrund der Abschattung die Einlassöffnung 46 nicht erreichen können.

Ebenso ist es möglich, dass aufgrund der Temperaturdifferenz im Inneren des Strömungsraums 22 im Verhältnis zur äußeren Umgebung des Strömungsraums 22 sich Kondenswasser an der Innenwand des Strömungsraums 22 bildet. Ebenfalls durch die Strömung des fluiden Mediums 12 wird dieses Kondenswasser, welches sich bevorzugt als Film an der Innenwandfläche des Strömungsraums 22 bildet, in Strömungsrichtung mitgefördert. Um zu verhindern, dass dieser Film aus Kondenswasser über die Einlassöffnungen 46 in den Messraum 40 eindringt, kann ferner, wie in Figur 1 gezeigt, eine Rippe 58 im Bereich der Einlassöffnung 46 vorgesehen sein, um einen die Außenfläche des Schutzrohrs 16 hinabgleitenden Film aus Kondenswasser abzufangen und über den durch die Rippe 58 gebildeten Ableitkanal 60 um den Bereich der Einlassöffnungen 46 herumzuleiten. Auf der strömungsabwärtigen Seite fungiert die Rippe 58 als Abrisskante 62, an der ein sich bildender Tropfen von dem strömenden fluiden Medium mitgerissen werden kann. Die Einlassöffnungen 46 sind weiterhin bevorzugt als Stutzen gebildet, um an ihrem unteren Ende eine weitere Abrisskante 62 zu bilden. Wie der Darstellung von Figur 1 weiter zu entnehmen ist, weist das Gehäuse 30 mindestens einen Elektronikraum 64 auf, welcher bevorzugt eine Ansteuerschaltung 66 der Sensorvorrichtung 10 aufnimmt. Das Feuchtemodul 32 ist folglich zumindest teilweise im Elektronikraum 64 als auch mit dem Sensorelement 42 und der Schutzmembran 44 im Messraum 40 oberhalb des Schutzrohrs 16 angeordnet.

Figur 3 zeigt eine Querschnittsdarstellung einer weiteren möglichen Ausführungsform der erfindungsgemäßen Sensorvorrichtung 10. Nachfolgend wird nunmehr lediglich auf die Unterschiede zu der in Fig. 1 gezeigten Ausführungsform beschrieben.

Wie in Fig. 3 gezeigt ist, weist das Schutzrohr 16, gegenüber der in Fig. 1 dargestellten Ausführungsform, eine geänderte Oberflächenkontur im Inneren des Strömungsraums 22 auf. Insbesondere sind an dem Schutzrohr 16 mehrere Abrisskanten 62a, 62b, 62c ausgebildet, wie nachstehend ausführlicher erläutert wird. Insbesondere weist das Schutzrohr 16 mindestens eine Abrisskante 62c in der Nähe der Einlassöffnung 46 und mindestens eine Verrundung 62d mit relativ großem Radius in der Nähe der Auslassöffnung 48 auf. Ein Kanten-Radius der Abrisskante 62c in der Nähe der Einlassöffnung 46 ist wesentlich kleiner oder auch scharfkantig als 90°-Winkel als ein Radius der Verrundung 62d in der Nähe der Auslassöffnung 48. So weist beispielsweise der Bereich des Schutzrohrs 16 zwischen den Einlassöffnungen 46 und der Außenfläche des Ausströmstutzens 18a, also einer Prallfläche 78b einen Verlauf auf, der zur Rotationsachse 14 hin unmittelbar an die Einlassöffnungen 46 angrenzt und eine umlaufende Prallfläche 78a ausbildet. Zur Bildung einer Abrisskante 62a am in der Darstellung unteren Ende der zuvor beschriebenen Prallfläche 78a ist zwischen dieser Abrisskante 62a und der Außenfläche des Ausströmstutzens 18a ein Spalt 80 vorgesehen. Die Ausgestaltung der unmittelbar an die Einlassöffnung 46 angrenzende Prallfläche 78a erhöht im Bereich der Einlassöffnung 46 das Überdruck-Niveau und schafft somit eine vorteilhafte erhöhte Durchströmung des Schutzrohrs 16. Ferner weist der in Hauptströmungsrichtung 12 betrachtet in Richtung der Auslassöffnung 48 herausragende Ausströmstutzen 18a auf seiner Außenfläche die weitere Prallfläche 78b auf. Am unteren Ende der Prallfläche 78b ist die Verrundung 62d ausgebildet, die den Übergang zwischen der Prallfläche 78b und der die Auslassöffnung 48 begrenzenden Wand des Ausströmstutzens 18a bildet. Der Radius der Verrundung 62d ist aufgrund der Notwendigkeit zur Erzeugung eines möglichst großen Unterdruckes an der Auslassöffnung 48 möglichst groß auszuführen. Die Abrisskante 62a kann einen vergleichsweisen großen Kanten-Radius aufweisen. Beispielsweise liegt der Radius der Verrundung 62d im Bereich von 2 bis 15 mm, beispielsweise 8 mm. Die Abrisskante 62a einen kann einen Kanten-Radius von 1 mm bis 10 mm auf, beispielsweise 5 mm.

Weiter bildet das Schutzrohr 16 in seinem äußeren Übergangsbereich zum Wandabschnitt 20 ebenfalls eine Prallfläche 78c aus, welche in einen radial nach innen anschließenden ebenen Verlauf übergeht. In diesem Bereich des Übergangs in den ebenen Verlauf ist eine weitere Abrisskante 62b gebildet, an welchem sich ein beispielsweise an der Innenoberfläche des Wandabschnitts 20 entlang fließender Wasserfilm sammeln kann und durch eine Tropfenbildung an der Abrisskante 62b abtropfen und von der Strömung des Mediums mitgerissen werden kann. Der Radius der Abrisskante 62b kann beispielsweise im Bereich 0,1 mm bis 5 mm liegen.

Die Einlassöffnungen 46 treten bevorzugt aus dem zuvor beschriebenen ebenen Verlauf des Schutzrohrs 16 heraus. Die somit gebildete weitere Abrisskante 62c am radial äußeren Abschnitt der Einlassöffnungen 46 kann bevorzugt einen Kanten-Radius im Bereich von ca. 0,1 mm bis 1,0 mm aufweisen, beispielsweise 0,8 mm, und somit einen Kanten-Radius, der deutlich kleiner als der Kanten-Radius der Abrisskanten 62a und 62b ist. Die Abrisskante 62b kann einen Kanten-Radius im Bereich von ca. 0,1 mm bis 5 mm aufweisen, beispielsweise 2 mm.

## Patentansprüche

1. Sensorvorrichtung (10) zur Erfassung einer Feuchte eines strömenden fluiden Mediums, umfassend mindestens ein Feuchtemodul (32) mit mindestens einem Sensorelement (42) zur Erfassung der Feuchte, wobei die Sensorvorrichtung (10) weiterhin mindestens ein Gehäuse (30) aufweist, wobei das Feuchtemodul (32) zumindest teilweise in einem Messraum (40) des Gehäuses (30) angeordnet ist,
wobei das Gehäuse (30) ein in einer axialen Richtung (34a) in das strömende fluide Medium ragendes Schutzrohr (16) aufweist, wobei das Schutzrohr (16) mindestens einen Einströmpfad (36) aufweist, wobei das fluide Medium durch den Einströmpfad (36) entgegen der axialen Richtung (34b) in den Messraum (40) einströmen kann, und wobei das Schutzrohr (16) weiterhin mindestens einen Ausströmpfad (38) aufweist, wobei das fluide Medium durch den Ausströmpfad (38) in axialer Richtung (34a) aus dem Messraum (40) ausströmen kann und wobei zwischen mindestens einer Mündung (50) des Einströmpfads (36) in den Messraum (40) und mindestens einer Mündung (52) des Ausströmpfads (38) in den Messraum (40) mindestens ein Konturabschnitt (54) angeordnet ist, **dadurch gekennzeichnet, dass** die Sensorvorrichtung mindestens eine das Senrorelement (42) abschirmende Schutzmembran (44) umfasst, wobei der mindestens eine Konturabschnitt (54) parallel zu der Schutzmembran (44) verläuft, und dass das Schutzrohr (16) einen Ausströmstutzen (18a) und mindestens einen Einlassstutzen (18b) umfasst, wobei der Ausströmpfad (38) zumindest teilweise in dem Ausströmstutzen (18a) angeordnet ist, wobei der Einströmpfad (36) in dem Einströmstutzen (18b) angeordnet ist, wobei das Schutzrohr (16) zwischen der äußeren Umfangsfläche des Ausströmstutzens (18a) und den Einlassöffnungen (46) einen Ringspalt (56) aufweist.

2. Sensorvorrichtung (10) nach einem der vorhergehenden Ansprüche, wobei der Einströmpfad (36) und der Ausströmpfad (38) rotationssymmetrisch zu einer in der axialen Richtung (34a) verlaufenden Rotationsachse (14) ausgebildet sind.

3. Sensorvorrichtung (10) nach einem der vorhergehenden Ansprüche, wobei der Einströmpfad (36) den Ausströmpfad (38) zumindest teilweise konzentrisch ringförmig umschließt.

4. Sensorvorrichtung (10) nach einem der vorhergehenden Ansprüche, wobei der Einströmpfad (36) mindestens eine Einlassöffnung (46) in dem Schutzrohr (16) aufweist, wobei der Ausströmpfad (38) mindestens eine Auslassöffnung (48) in dem Schutzrohr (16) aufweist, wobei das Schutzrohr (16) derart in eine Strömung des fluiden Mediums hineinragt, dass die Auslassöffnung (48) weiter im Inneren der Strömung angeordnet ist als die Einlassöffnung (46).

5. Sensorvorrichtung (10) nach dem vorhergehenden Anspruch, wobei das Schutzrohr (16) mindestens eine Abrisskante (62c) in der Nähe der Einlassöffnung (46) und mindestens eine Verrundung (62d) in der Nähe der Auslassöffnung (48) aufweist, wobei ein Kanten-Radius der Abrisskante (62c) in der Nähe der Einlassöffnung (46) deutlich kleiner als ein Radius der Verrundung (62d) in der Nähe der Auslassöffnung (48) ist.

6. Sensorvorrichtung (10) nach einem der beiden vorhergehenden Ansprüche, wobei eine Mehrzahl von konzentrisch um die Auslassöffnung (48) radial beabstandet angeordneten Einlassöffnungen (46) vorgesehen ist.

7. Sensorvorrichtung (10) nach einem der vorhergehenden Ansprüche, wobei das Gehäuse (30) mindestens ein Befestigungselement (24) zum Befestigen des Gehäuses (30) an einem Wandabschnitt (20) eines von dem fluiden Medium durchströmten Strömungsraums (22) aufweist, wobei das Befestigungselement (24) derart ausgestaltet ist, dass in befestigtem Zustand das Schutzrohr (16) zumindest teilweise in den Strömungsraum (22) hineinragt.

## Claims

1. Sensor device (10) for detecting a moisture content of a flowing fluid medium, comprising at least one moisture module (32) having at least one sensor element (42) for recording the moisture content, the sensor device (10) also having at least one housing (30), the moisture module (32) being at least partially arranged in a measuring chamber (40) of the housing (30),
the housing (30) having a protective tube (16), protruding in an axial direction (34a) into the flowing fluid medium, the protective tube. (16) having at least one inflow path (36), the fluid medium being able to flow through the inflow path (36) counter to the axial direction (34b) into the measuring chamber (40), and the protective tube (16) also having at least one outflow path (38), the fluid medium being able to flow through the outflow path (38) in the axial direction (34a) out of the measuring chamber (40), and at least one contour section (54) being arranged between at least one mouth (50) of the inflow path (36) into the measuring chamber (40) and at least one mouth (52) of the outflow path (38) into the measuring chamber (40), **characterized in that** the sensor device comprises at least one protective membrane (44) shielding the sensor element (42), the at least one contour section (54) running parallel to the protective membrane (44), and **in that** the protective tube (16) comprises an outflow nozzle (18a) and at least one inlet nozzle (18b), the outflow path (38) being at least partially arranged in the outflow nozzle (18a), the inflow path (36) being arranged in the inflow nozzle (18b), the protective tube (16) having an annular gap (56) between the outer circumferential surface of the outflow nozzle (18a) and the inlet openings (46).

2. Sensor device (10) according to one of the preceding claims, the inflow path (36) and the outflow path (38) being formed rotationally symmetrically in relation to a rotation axis (14) running in the axial direction (34a).

3. Sensor device (10) according to either of the preceding claims, the inflow path (36) enclosing the outflow path (38) in an at least partially concentrically annular manner.

4. Sensor device (10) according to one of the preceding claims, the inflow path (36) having at least one inlet opening (46) in the protective tube (16), the outflow path (38) having at least one outlet opening (48) in the protective tube (16), the protective tube (16) protruding into a flow of the fluid medium in such a way that the outlet opening (48) is arranged further within the flow than the inlet opening (46).

5. Sensor device (10) according to the preceding claim, the protective tube (16) having at least one break-away edge (62c) in the vicinity of the inlet opening (46) and at least one rounding (62d) in the vicinity of the outlet opening (48), an edge radius of the break-away edge (62c) in the vicinity of the inlet opening (46) being significantly smaller than a radius of the rounding (62d) in the vicinity of the outlet opening (48).

6. Sensor device (10) according to one of the two preceding claims, a plurality of inlet openings (46) being provided, arranged radially spaced apart concentrically around the outlet opening (48).

7. Sensor device (10) according to one of the preceding claims, the housing (30) having at least one fastening element (24) for fastening the housing (30) on a wall section (20) of a flow chamber (22) through which the fluid medium flows, the fastening element (24) being designed in such a way that, in the fastened state, the protective tube (16) protrudes at least partially into the flow chamber (22).

## Revendications

1. Dispositif capteur (10) pour la détection d'une humidité d'un milieu fluide en écoulement, comprenant au moins un module d'humidité (32) muni d'au moins un élément capteur (42) pour la détection de l'humidité, le dispositif capteur (10) comprenant en outre au moins un boîtier (30), le module d'humidité (32) étant au moins partiellement agencé dans une chambre de mesure (40) du boîtier (30),
le boîtier (30) comprenant un tube de protection (16) faisant saillie dans une direction axiale (34a) dans le milieu fluide en écoulement, le tube de protection (16) comprenant au moins une voie d'entrée (36), le milieu fluide pouvant entrer dans la chambre de mesure (40) par la voie d'entrée (36) à l'encontre de la direction axiale (34b), et le tube de protection (16) comprenant en outre au moins une voie de sortie (38), le milieu fluide pouvant sortir de la chambre de mesure (40) par la voie de sortie (38) dans la direction axiale (34a), et au moins une section de contour (54) étant agencée entre au moins une embouchure (50) de la voie d'entrée (36) dans la chambre de mesure (40) et au moins une embouchure (52) de la voie de sortie (38) dans la chambre de mesure (40), **caractérisé en ce que** le dispositif capteur comprend au moins une membrane de protection (44) couvrant l'élément capteur (42), l'au moins une section de contour (54) s'étendant parallèlement à la membrane de protection (44), et **en ce que** le tube de protection (16) comprend une tubulure de sortie (18a) et au moins une tubulure d'admission (18b), la voie de sortie (38) étant agencée au moins partiellement dans la tubulure de sortie (18a), la voie d'entrée (36) étant agencée dans la tubulure d'entrée (18b), le tube de protection (16) comprenant un espace annulaire (56) entre la surface circonférentielle extérieure de la tubulure de sortie (18a) et les ouvertures d'entrée (46).

2. Dispositif capteur (10) selon l'une quelconque des revendications précédentes, la voie d'entrée (36) et la voie de sortie (38) étant configurées sous forme symétrique par rotation par rapport à un axe de rotation (14) s'étendant dans la direction axiale (34a).

3. Dispositif capteur (10) selon l'une quelconque des revendications précédentes, la voie d'entrée (36) entourant au moins partiellement la voie de sortie (38) de manière concentrique sous forme annulaire.

4. Dispositif capteur (10) selon l'une quelconque des revendications précédentes, la voie d'entrée (36) comprenant au moins une ouverture d'entrée (46) dans le tube de protection (16), la voie de sortie (38) comprenant au moins une ouverture de sortie (48) dans le tube de protection (16), le tube de protection (16) faisant saillie dans un écoulement du milieu fluide de telle sorte que l'ouverture de sortie (48) est agencée plus loin à l'intérieur de l'écoulement que l'ouverture d'entrée (46).

5. Dispositif capteur (10) selon la revendication précédente, le tube de protection (16) comprenant au moins une arête de rupture (62c) à proximité de l'ouverture d'entrée (46) et au moins un arrondissement (62d) à proximité de l'ouverture de sortie (48), un rayon d'arête de l'arête de rupture (62c) à proximité de l'ouverture d'entrée (46) étant considérablement plus petit qu'un rayon de l'arrondissement (62d) à proximité de l'ouverture de sortie (48).

6. Dispositif capteur (10) selon l'une quelconque des deux revendications précédentes, une pluralité d'ouvertures d'entrée (46) agencées de manière espacée radialement concentriquement autour de l'ouverture de sortie (48) étant prévue.

7. Dispositif capteur (10) selon l'une quelconque des revendications précédentes, le boîtier (30) comprenant au moins un élément de fixation (24) pour la fixation du boîtier (30) à une section de paroi (20) d'une chambre d'écoulement (22) traversée par le milieu fluide, l'élément de fixation (24) étant conçu de telle sorte qu'à l'état fixé, le tube de protection (16) fait au moins partiellement saillie dans la chambre d'écoulement (22).
